# EUROPEAN PATENT APPLICATION

(11) **EP 4 089 095 A1**
(43) Date of publication of application: **16.11.2022**
(21) Application number: 21738457.7
(22) Date of filing: 07.01.2021
(51) Int. Cl.: C07D 495/04, A61K 31/4365, A61K 31/444, A61P 35/00

(54) **DEUTERATED THIENOPYRIDINE COMPOUND**

(30) Priority: 07.01.2020 CN 202010015514; 22.04.2020 CN 202010323048; 08.05.2020 CN 202010389596
(71) Applicant: Medshine Discovery Inc., Nanjing, Jiangsu 210032 (CN)
(72) Inventor: FU, Zhifei, Shanghai 200131 (CN); LUO, Miaorong, Shanghai 200131 (CN); ZHANG, Yang, Shanghai 200131 (CN); LI, Jian, Shanghai 200131 (CN); CHEN, Shuhui, Shanghai 200131 (CN)
(74) Representative: Dehns
(86) International application number: PCT/CN2021/070646
(87) International publication number: WO 2021/139717

(57) **Abstract**

Disclosed is a deuterated thienopyridine compound. Specifically disclosed are a compound represented by formula (I) and a pharmaceutically acceptable salt thereof.

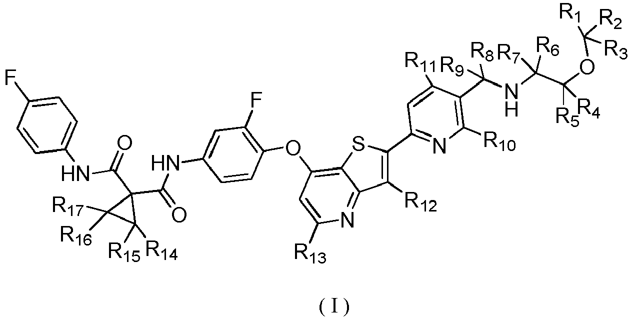

## Description

**The present application claims the right of the following priorities for:**
CN202010015514.X, filed on January 7, 2020;
CN202010323048.1, filed on April 22, 2020;
CN202010389596.4 filed on May 8, 2020.

### TECHNICAL FIELD

The present disclosure relates to a series of deuterated thienopyridine compounds, in particular to a compound represented by formula (I) and a pharmaceutically acceptable salt.

### BACKGROUND

The present disclosure is a multi-receptor tyrosine kinase inhibitor, targeting RET, MET, VEGFR-1, -2, -3, KIT, TRKB, FLT-3, AXL, TIE-2, etc. Tyrosine kinase plays a very important role in the occurrence and development of tumors. Drug research and development targeting tyrosine kinase has become a hot spot in the international antitumor drug research. Tyrosine kinase inhibitor achieves anti-tumor effects by inhibiting the damage and repair of tumor cells, causing cell division to stay in the G1 phase, inducing and maintaining apoptosis, and anti-angiogenesis.

The present disclosure relates to compounds that inhibit the activity of protein tyrosine kinase. Tyrosine kinase can be classified as growth factor receptor (e.g., EGFR, PDGFR, FGFR, and erbB2) or non-receptor (e.g., c-src and bcr-abl) kinase. Receptor-type tyrosine kinase includes about 20 different subfamilies. Non-receptor type tyrosine kinase includes many subfamilies. These tyrosine kinases have different biological activities. Receptor tyrosine kinase is a huge enzyme that transmembrane and has extracellular binding domain for growth factor, transmembrane domain, and intracellular group that function as kinase to phosphorylate specific tyrosine residues in protein, and therefore affect cell proliferation. Inappropriate or unsuitable protein kinase activity can produce disease states associated with these inappropriate kinase activities.

Mirati has developed Sitravatinib, a kinase inhibitor as described above, structured as in Formula II, which has demonstrated good efficacy in the clinic, but due to its lack of stability in humans and high dose administration, there is a need to develop more stable and reliable alternatives to achieve equal or better efficacy at lower dose administration.

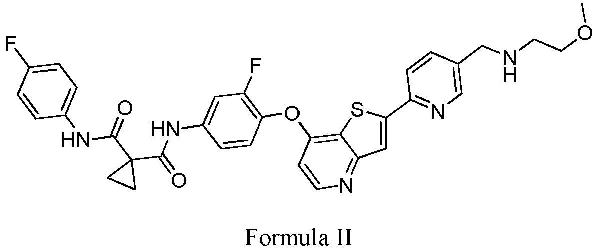

### CONTENT OF THE PRESENT INVENTION

The present disclosure provides a compound represented by formula (I) or a pharmaceutically acceptable salt thereof, 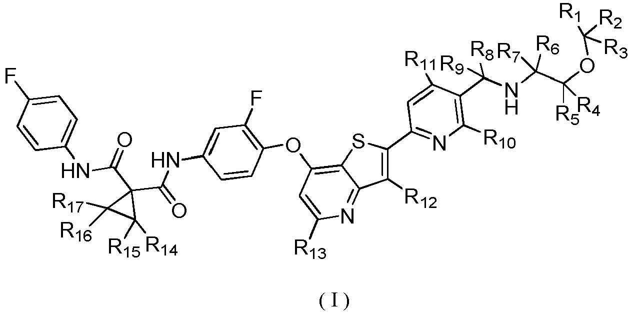 wherein,
R₁, R₂, R₃, R₄, R₅, R₆, R₇, R₈, R₉, R₁₀, R₁₁, R₁₂, R₁₃, R₁₄, R₁₅, R₁₆ and R₁₇ are each independently selected from H and D, and at least one of them is D.

In some embodiments of the present disclosure, R₁, R₂ and R₃ are each independently selected from H and D, and other variables are as defined in the present disclosure.

In some embodiments of the present disclosure, R₄ and R₅ are each independently selected from H and D, and other variables are as defined in the present disclosure.

In some embodiments of the present disclosure, R₆ and R₇ are each independently selected from H and D, and other variables are as defined in the present disclosure.

In some embodiments of the present disclosure, R₈ and R₉ are each independently selected from H and D, and other variables are as defined in the present disclosure.

In some embodiments of the present disclosure, R₁₀ is selected from H and D, and other variables are as defined in the present disclosure.

In some embodiments of the present disclosure, R₁₁ is selected from H and D, and other variables are as defined in the present disclosure.

In some embodiments of the present disclosure, R₁₂ is selected from H and D, and other variables are as defined in the present disclosure.

In some embodiments of the present disclosure, R₁₃ is selected from H and D, and other variables are as defined in the present disclosure.

In some embodiments of the present disclosure, R₁₄, R₁₅, R₁₆ and R₁₇ are each independently selected from H and D, and other variables are as defined in the present disclosure.

In some embodiments of the present disclosure, the compound or the pharmaceutically acceptable salt thereof is selected from 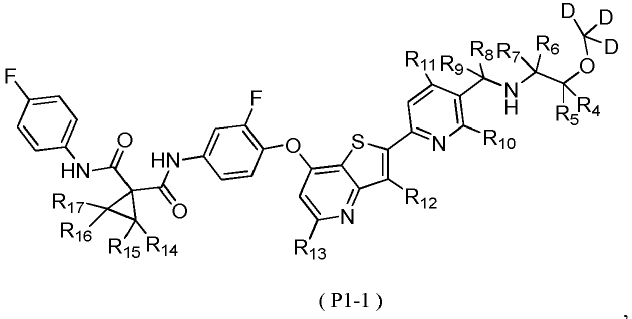 wherein, R₄, R₅, R₆, R₇, R₈, R₉, R₁₀, R₁₁, R₁₂, R₁₃, R₁₄, R₁₅, R₁₆ and R₁₇ are as defined in the present disclosure.

The present disclosure provides the following compounds or pharmaceutically acceptable salts thereof,

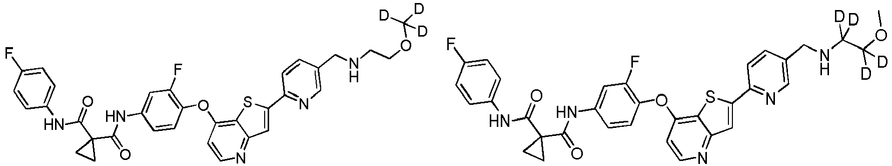

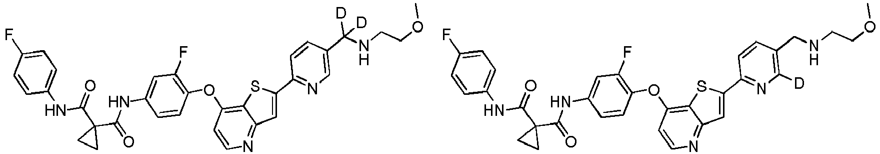

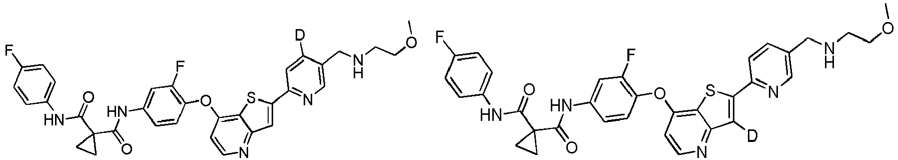

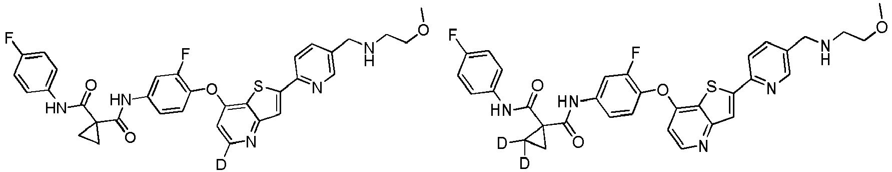

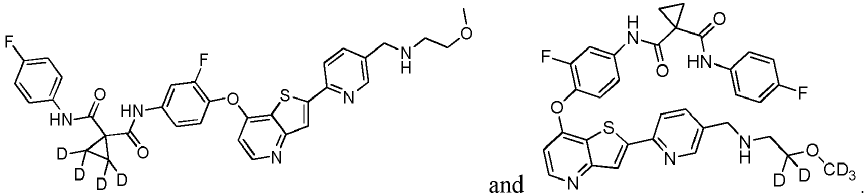

The present disclosure also provides use of the compound or the pharmaceutically acceptable salt thereof in the manufacture of drugs for diseases associated with tyrosine kinase.

### Technical effects

The compound of the present disclosure has good inhibitory effect on Axl, c-Kit, Mer, DDR2, VEGFR1, VEGFR2, VEGFR3, TrkA and FLT3, better hepatic microsomal metabolic stability and superior pharmacokinetic properties.

### Definition and description

Unless otherwise stated, the following terms and phrases used herein have the following meanings. A specific term or phrase should not be considered indefinite or unclear in the absence of a particular definition, but should be understood according to the common meaning. When a trade name appears herein, it is intended to refer to its corresponding commercial product or active ingredient thereof.

The term "pharmaceutically acceptable" is used herein in terms of those compounds, materials, compositions, and/or dosage forms, which are suitable for use in contact with human and animal tissues within the scope of reliable medical judgment, without excessive toxicity, irritation, anaphylactic reaction or other problems or complications, commensurate with a reasonable benefit/risk ratio.

The term "pharmaceutically acceptable salt" refers to a salt of the compound of the present disclosure that is prepared by reacting the compound having a specific substituent of the present disclosure with a relatively non-toxic acid or base. When the compound of the present disclosure contains a relatively acidic functional group, a base addition salt can be obtained by contacting the neutral form of the compound with a sufficient amount of a base in a pure solution or a suitable inert solvent. The pharmaceutically acceptable base addition salt includes a salt of sodium, potassium, calcium, ammonium, organic amine or magnesium, or similar salts. When the compound of the present disclosure contains a relatively basic functional group, an acid addition salt can be obtained by contacting the neutral form of the compound with a sufficient amount of an acid in a pure solution or a suitable inert solvent. Examples of the pharmaceutically acceptable acid addition salts include salts derived from inorganic acids, such as hydrochloric acid, hydrobromic acid, nitric acid, carbonic acid, bicarbonate, phosphoric acid, monohydrogen phosphate, dihydrogen phosphate, sulfuric acid, hydrogen sulfate, hydroiodic acid, phosphorous acid, and the like; and an organic acid salt, wherein the organic acid includes, for example, acetic acid, propionic acid, isobutyric acid, maleic acid, malonic acid, benzoic acid, succinic acid, suberic acid, fumaric acid, lactic acid, mandelic acid, phthalic acid, benzenesulfonic acid, p-toluenesulfonic acid, citric acid, tartaric acid, and methanesulfonic acid, and the like; and salts of amino acid (such as arginine and the like), and a salt of an organic acid such as glucuronic acid and the like. Certain specific compounds of the present disclosure contain both basic and acidic functional groups, thus can be converted to either base or acid addition salts.

The pharmaceutically acceptable salt of the present disclosure can be prepared from the parent compound that contains an acidic or basic group by conventional chemical methods. Generally, such salt can be prepared by reacting the free acid or base form of the compound with a stoichiometric amount of an appropriate base or acid in water or an organic solvent or a mixture thereof.

The compounds of the present disclosure may exist in specific geometric or stereoisomeric forms. The present disclosure contemplates all such compounds, including cis and trans isomers, (-)-and (+)-enantiomers, (*R*)-and (*S*)-enantiomers, diastereoisomers, (*D*)-isomers, (*L*)-isomers, and racemic and other mixtures thereof, such as enantiomers or diastereomer enriched mixtures, all of which are within the scope of the present disclosure. Additional asymmetric carbon atoms may be present in substituents such as alkyl. All these isomers and their mixtures are encompassed within the scope of the present disclosure.

The term "optional" or "optionally" means that the subsequently described event or circumstance may, but does not necessarily, occur, and the description includes instances where the event or circumstance occurs and instances where it does not.

The term "substituted" means one or more than one hydrogen atom(s) on a specific atom is substituted with the substituent, including deuterium and hydrogen variables, as long as the valence of the specific atom is normal and the substituted compound is stable. When the substituent is an oxygen (i.e., =O), it means two hydrogen atoms are substituted. Positions on an aromatic ring cannot be substituted with a ketone. The term "optionally substituted" means an atom can be substituted with a substituent or not, unless otherwise specified, the type and number of the substituent may be arbitrary as long as being chemically achievable.

When any variable (such as R) occurs in the constitution or structure of the compound more than once, the definition of the variable at each occurrence is independent. Thus, for example, if a group is substituted with 0-2 R, the group can be optionally substituted with up to two R, wherein the definition of R at each occurrence is independent. Moreover, a combination of the substituent and/or the variant thereof is allowed only when the combination results in a stable compound.

Unless otherwise specified, D as described in the present disclosure represents deuterium (²H).

Unless otherwise specified, when a group has one or more linkable sites, any one or more sites of the group can be linked to other groups through chemical bonds. When the linking site of the chemical bond is not positioned, and there is H atom at the linkable site, then the number of H atom at the site will decrease correspondingly with the number of chemical bond linking thereto so as to meet the corresponding valence. The chemical bond between the site and other groups can be represented by a straight solid bond ( 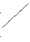 ), a straight dashed bond 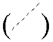 or a wavy line 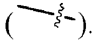 For example, the straight solid bond in -OCH₃ means that it is linked to other groups through the oxygen atom in the group; the straight dashed bonds in 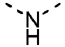 means that it is linked to other groups through the two ends of nitrogen atom in the group; the wave lines in 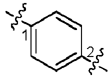 means that the phenyl group is linked to other groups through carbon atoms at position 1 and position 2; 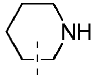 means that it can be linked to other groups through any linkable sites on the piperidinyl by one chemical bond, including at least four types of linkage, including 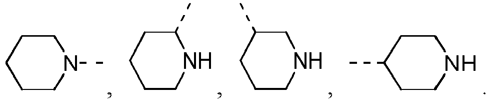 Even though the H atom is drawn on the -N-, 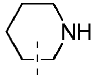 still includes the linkage of 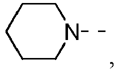 merely when one chemical bond was connected, the H of this site will be reduced by one to the corresponding monovalent piperidinyl.

The compounds of the present disclosure can be prepared by a variety of synthetic methods known to those skilled in the art, including the specific embodiments listed below, the embodiments formed by their combination with other chemical synthesis methods, and equivalent alternatives known to those skilled in the art, preferred implementations include but are not limited to the embodiments of the present disclosure.

The structure of the compounds of the present disclosure can be confirmed by conventional methods known to those skilled in the art, and if the present disclosure involves an absolute configuration of a compound, then the absolute configuration can be confirmed by means of conventional means in the art. For example, in the case of single crystal X-ray diffraction (SXRD), the absolute configuration can be confirmed by collecting diffraction intensity data from the cultured single crystal using a Bruker D8 venture diffractometer with CuKα radiation as the light source and scanning mode: ϕ/ω scan, and after collecting the relevant data, the crystal structure can be further analyzed by direct method (Shelxs97).

The solvent used in the present disclosure is commercially available.

The compounds of the present disclosure are named according to the conventional naming principles in the art or by ChemDraw^{®} software, and the commercially available compounds use the supplier catalog names.

### DETAILED DESCRIPTION OF THE PREFERRED EMBODIMENT

The present disclosure is described in detail by the embodiments below, but it does not mean that there are any adverse restrictions on the present disclosure. The present disclosure has been described in detail herein, wherein specific embodiments thereof are also disclosed, and it will be apparent to those skilled in the art that various variations and improvements can be made to specific embodiments of the present disclosure without departing from the spirit and scope of the present disclosure.

### Embodiment 1

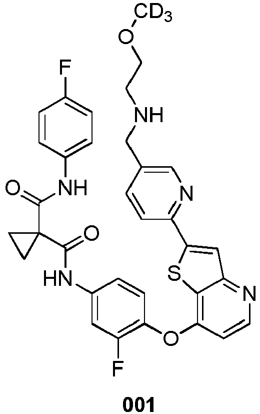

Synthetic route:

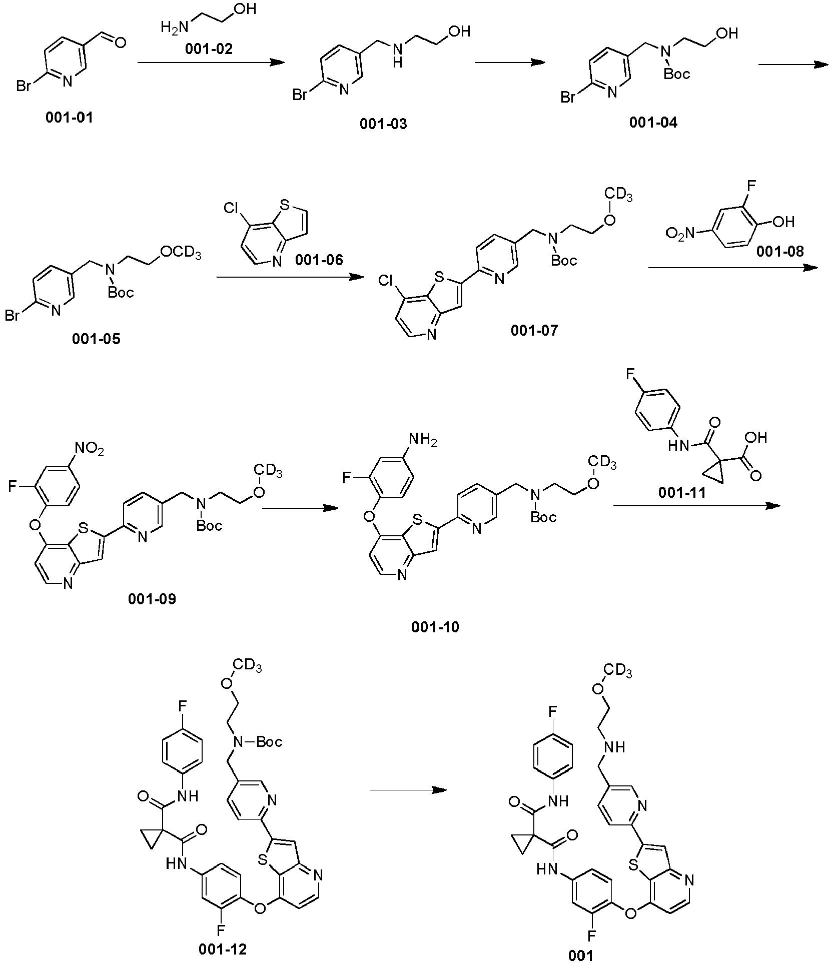

### Step 1: Synthesis of compound 001-03

Compound 001-01 (20 g, 107.52 mmol, 1 eq) and compound 001-02 (7.88 g, 129.03 mmol, 7.80 mL, 1.2 eq) were dissolved in dichloromethane (200 mL), sodium borohydride acetate (29.63 g, 139.78 mmol, 1.3 eq) was added at 20 °C, the reaction was continued for 1.5 hours, and the compound 001-03 was obtained by concentrating to dryness.

LCMS: MS (ESI) m/z: 231.1 [M+1]⁺.

### Step 2: Synthesis of compound 001-04

Compound 001-03 (24.85 g, 107.53 mmol, 1 eq) was dissolved in dichloromethane (200 mL), triethylamine (38.08 g, 376.37 mmol, 52.39 mL, 3.5 eq) was added, and then di-tert-butyl dicarbonate (28.16 g, 129.04 mmol, 29.64 mL, 1.2 eq) was added, and the reaction was continued at 20 °C for 1 hour. The reaction solution was concentrated to dryness, ethyl acetate (100 mL) and water (100 mL) were added to dissolve and dilute, and the organic phase was separated, washed with water (100 mL), washed with saturated sodium chloride solution (100 mL), dried with anhydrous sodium sulfate and concentrated. The crude product was separated by silica gel column chromatography (120 g flash column, ethyl acetate in petroleum ether: 0-100%) to obtain compound 001-04.
LCMS: MS (ESI) m/z: 331.1[M+1]⁺;
¹H NMR (400 MHz, CDCl₃) δ 1.45 (br s, 9H), 3.27-3.41 (m, 2H), 3.73 (br s, 2H), 4.46 (br s, 2H), 7.47 (br s, 2H), 8.27 (br s, 1H).

### Step 3: Synthesis of compound 001-05

Compound 001-04 (22 g, 66.42 mmol, 1 eq) was dissolved in tetrahydrofuran (250 mL) and dimethylformamide (44 mL), cesium carbonate (32.46 g, 99.64 mmol, 1.5 eq) was added, and then deuterated iodomethane (14.44 g, 99.64 mmol, 6.20 mL, 1.5 eq) was added, and the reaction was carried out at 35 °C for 63 hours. The reaction solution was filtered while hot and concentrated. The crude product was separated by silica gel column chromatography (120 g flash column, ethyl acetate in petroleum ether: 0- 30%) to obtain compound 001-05.

LCMS: MS (ESI) m/z: 348.1[M+1]⁺.

### Step 4: Synthesis of compound 001-07

Compound 001-06 (10.39 g, 61.28 mmol, 2 eq) was dissolved in tetrahydrofuran (150 mL), the solution was cooled to -78 °C in an dry ice ethanol bath, then n-butyllithium (2.5 M, 24.51 mL, 2 eq) was slowly added dropwise, the solution was continued stirring for 0.5 hours after dropping, then zinc chloride (2 M, 30.64 mL, 2 eq) was added, and the solution was moved to react at 20 °C for 1 hour. Then compound 001-05 (10.67 g, 30.64 mmol, 1 eq) and tetrakis (triphenylphosphine) palladium (1.06 g, 919.17 µmol, 0.03 eq) in tetrahydrofuran (100 mL) were added and moved to react at 70 °C for 1 hour. Saturated sodium bicarbonate solution (20 mL) was added to the reaction solution, then ethyl acetate (100 mL) was added, the organic phase was separated, washed with saturated sodium chloride solution (150 mL), dried with anhydrous sodium sulfate and concentrated. The crude product was separated by silica gel column chromatography (120 g flash column, ethyl acetate in petroleum ether: 0- 80%) to obtain compound 001-07. LCMS: MS (ESI) m/z: 437.0[M+1]⁺;
^{I}H NMR (400 MHz, CDCl₃) δ 1.49 (br d, J=19.58 Hz, 9H), 3.23-3.72 (m, 4H), 4.59 (br s, 2H), 7.29 (d, J=5.02 Hz, 1H), 7.62-7.80 (m, 1H), 7.86 (d, J=8.28 Hz, 1H), 7.99 (s, 1H), 8.52-8.62 (m, 2H).

### Step 5: Synthesis of compound 001-09

Compound 001-08 (719.04 mg, 4.58 mmol, 2 eq) and compound 001-07 (1 g, 2.29 mmol, 1 eq) were dissolved in chlorobenzene (5 mL), and then diisopropylethylamine (739.43 mg, 5.72 mmol, 996.53 µL, 2.5 eq) was added, the reaction was carried out at 140 °C for 40 hours. The reaction solution was concentrated. The crude product was separated by silica gel column chromatography (ethyl acetate in petroleum ether: 0- 60%) to obtain compound 001-09.

LCMS: MS (ESI) m/z: 558.2[M+1]⁺;

^{I}H NMR (400 MHz, CDCl₃) δ 1.48 (br d, J=13.55 Hz, 9H), 3.27-3.61 (m, 4H), 4.58 (br s, 2H), 6.71 (d, J=5.27 Hz, 1H), 7.35 (t, J=8.28 Hz, 1H), 7.62-7.76 (m, 1H), 7.86 (d, J=8.03 Hz, 1H), 8.01 (s, 1H), 8.09-8.22 (m, 2H), 8.53 (s, 1H), 8.61 (d, J=5.27 Hz, 1H).

### Step 6: Synthesis of compound 001-10

Compound 001-09 (200 mg, 358.68 µmol, 1 eq) was dissolved in ethanol (4 mL) and water (2 mL), and then iron powder (60.09 mg, 1.08 mmol, 3 eq) and ammonium chloride (19.19 mg, 358.68 µmol, 1 eq) were added, the reaction was carried out at 70 °C for 3 hours. The reaction solution was filtered while hot to remove insolubles, the mother liquor was concentrated and dissolved in dichloromethane (20 mL), anhydrous sodium sulfate was added, insolubles were filtered out, and compound 001-10 was obtained by concentration.

LCMS: MS (ESI) m/z: 528.2 [M+1]⁺.

### Step 7: Synthesis of compound 001-12

Compound 001-10 (176 mg, 333.57 µmol, 1 eq) and compound 001-11 (96.79 mg, 433.64 µmol, 1.3 eq) were dissolved in dichloromethane (4 mL), and then diisopropylethylamine (86.22 mg, 667.14 µmol, 116.20 µL, 2 eq) and O-(7-azabenzotriazole-1-yl)-N,N,N,N-tetramethyl uronium hexafluorophosphate (177.57 mg, 467.00 µmol, 1.4 eq) were added, the reaction was carried out at 39°C for 6 hours. Water (20 mL) was added to the reaction solution, the organic phase was separated, washed with saturated bicarbonate sodium solution (30 mL), washed with saturated sodium chloride solution (30 mL) and concentrated. The crude product was separated by silica gel column chromatography (4 g flash column, methanol in dichloromethane: 0- 6%) to obtain compound 001-12.

LCMS: MS (ESI) m/z: 733.3[M+1]⁺.

### Step 8: Synthesis of compound 001

Compound 001-12 (196 mg, 267.46 µmol, 1 eq) was dissolved in dichloromethane (5 mL), and then trifluoroacetic acid (3.08 g, 27.01 mmol, 2 mL, 100.99 eq) was added, the reaction was carried out at 20 °C for 1 hour, and the reaction solution was concentrated to dryness. The crude product was separated by high performance liquid chromatography (column: Boston Green ODS 150^{∗}30 mm^{∗}5 µm; mobile phase: [water (0.075% trifluoroacetic acid)-acetonitrile]; acetonitrile %: 22%-52%, 8 min) to obtain the trifluoroacetate salt of the target compound 001.

LCMS: MS (ESI) m/z: 633.2[M+1]⁺;

¹H NMR (400 MHz, CD₃OD) δ 1.66 (m, 4H), 3.33 (br s, 2H), 3.66-3.74 (m, 2H), 4.37 (s, 2H), 6.88 (d, J=5.77 Hz, 1H), 7.03-7.13 (m, 2H), 7.40-7.50 (m, 2H), 7.53- 7.60 (m, 2H), 7.90 (dd, J=12.67, 2.13 Hz, 1H), 8.10 (dd, J=8.16, 2.13 Hz, 1H), 8.22-8.30 (m, 2H), 8.62 (br d, J=5.52 Hz, 1H), 8.76 (d, J=1.51 Hz, 1H).

### Embodiment 2

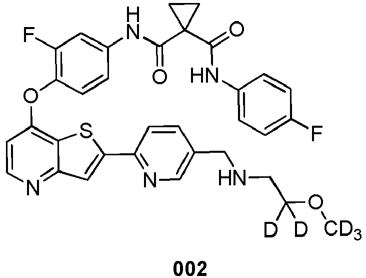

Synthetic route:

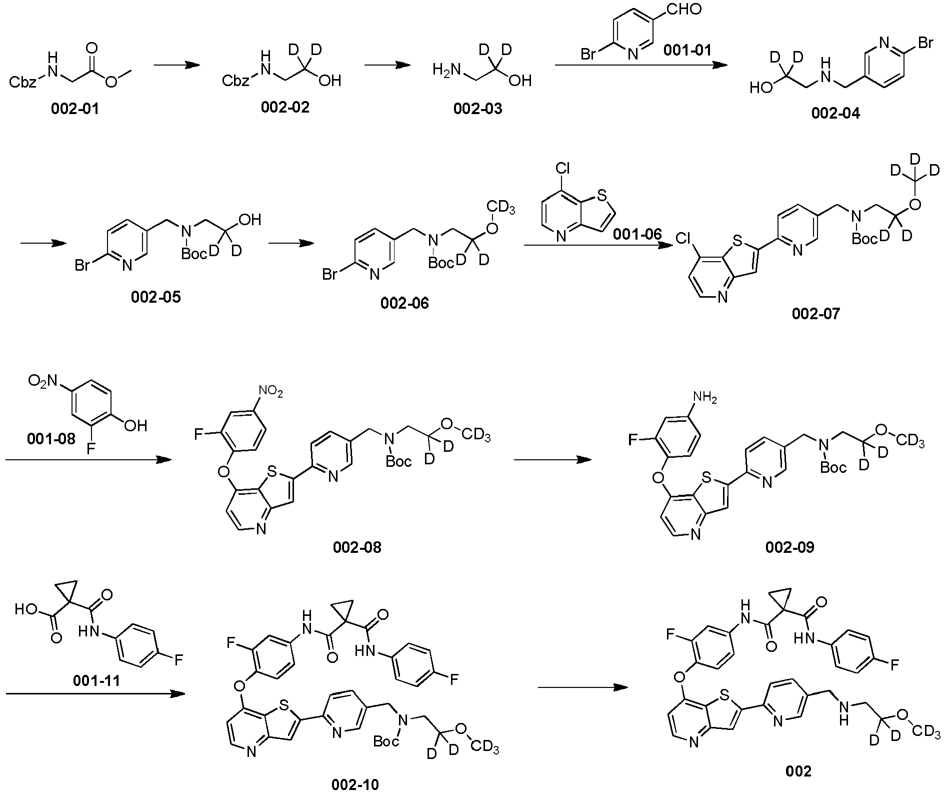

### Step 1: Synthesis of compound 002-02

002-01 (18 g, 80.64 mmol, 1 eq), deuterated methanol (50 mL) and deuterated sodium borohydride (9.76 g, 258.04 mmol, 3.2 eq) were added to a 100 mL bottle and stirred at 25 °C for 2 hours. Saturated ammonium chloride (50 mL) was added into the reaction solution, and then extracted by ethyl acetate (100 mL), and the filtrate of the three extractions were combined and concentrated, washed with saturated brine (100 mL×3) and concentrated to obtain compound 002-2.

LCMS: MS (ESI) m/z: 197.9[M+1]⁺.

### Step 2: Synthesis of compound 002-03

Compound 002-2 (5.1 g, 25.86 mmol, 1 eq), ethanol (20 mL) and palladium carbon (500 mg, 10% purity, 1.00 eq) were added to a 100 mL bottle, hydrogen was pumped three times, the solution was heated and stirred at 15 psi, 40°C for 48 hours. The palladium carbon was removed by filtration, and the reaction solution was concentrated to obtain compound 002-03.

¹H NMR (400 MHz, CD₃OD) δ ppm 2.76 (s, 2 H).

### Step 3: Synthesis of compound 002-04

002-03 (1.26 g, 19.97 mmol, 2.80 mL, 1.0 eq), 001-01 (3.71 g, 19.97 mmol, 1 eq), 1,2-dichloroethane (2 mL) were added to a 100 mL bottle. After stirring for 20 minutes, sodium borohydride acetate (4.87 g, 22.97 mmol, 1.15 eq) was added and stirred at 25 °C for 16 hours to obtain a solution of compound 002-04, which was used in the next step directly without separation.

LCMS: MS (ESI) m/z: 232.9[M+1]⁺.

### Step 4: Synthesis of compound 002-05

Triethylamine (12.11 g, 119.69 mmol, 16.66 mL, 6.0 eq), di-tert-butyl dicarbonate (13.06 g, 59.85 mmol, 13.75 mL, 3.0 eq) were added to the solution of compound 002-04 in the previous step and stirred at 25 °C for 3 hours. The reaction solution was evaporated to dryness and separated by column chromatography (20-100% ethyl acetate in petroleum ether) to obtain compound 002-05.

LCMS: MS (ESI) m/z: 333.1[M+1]⁺.

### Step 5: Synthesis of compound 002-06

Compound 002-05 (1.5 g, 4.50 mmol, 1 eq), N, N-dimethylformamide (10 mL), sodium hydride (235.88 mg, 5.90 mmol, 60% content, 1.31 eq) were added under nitrogen atmosphere at 0 °C and stirred for 30 minutes, and then deuterated iodomethane (1.28 g, 9.00 mmol, 560.48 µL, 2.0 eq) was added and stirred at 25 °C for 2 hours. Saturated ammonium chloride was added to the reaction solution, and then ethyl acetate (20 mL×3) was added for extraction, the organic phases were combined and concentrated. The concentrate was separated by column chromatography (20-100% ethyl acetate in petroleum ether) to obtain compound 002-06.

LCMS: MS (ESI) m/z: 350.1[M+1]⁺.

### Step 6: Synthesis of compound 002-07

Compound 002-06 (799.09 mg, 4.71 mmol, 3.0 eq) and tetrahydrofuran (8 mL) were added in a 100 mL three-neck flask under nitrogen atmosphere. After cooling to -78 °C, n-butyllithium (2.5 M, 1.88 mL, 3.0 eq) was added dropwise. After stirring for 30 minutes, a solution of zinc dichloride in 2-methyltetrahydrofuran (2 M, 2.36 mL, 3.0 eq) was added, the temperature was slowly raised to 25 °C, tetrakistriphenylphosphine palladium (181.45 mg, 157.03 µmol, 0.1eq) and a solution of compound 001-06 (550 mg, 1.57 mmol, 1 eq) in tetrahydrofuran (8 mL) were added after stirring for 1 hour. Then the reaction solution was refluxed at 70 °C for 1 hour. Saturated ammonium chloride (10 mL) was added to the reaction solution, and then ethyl acetate (10 mL×3) was added for extraction, the organic phases were combined and concentrated. The concentrate was separated by column chromatography (0-50% dichloromethane in ethyl acetate) to obtain compound 002-07.

LCMS: MS (ESI) m/z: 439.0[M+1]⁺.

### Step 7: Synthesis of compound 002-08

Compound 002-07 (474 mg, 1.08 mmol, 1 eq), chlorobenzene (5 mL), diisopropylamine (348.88 mg, 2.70 mmol, 470.19 µL, 2.5 eq) and compound 001-08 (339.26 mg, 2.16 mmol, 2.0 eq) were added to a 50 mL bottle and stirred at 140 °C for 72 hours. The reaction solution was concentrated and separated by column chromatography (dichloromethane : ethyl acetate = 20%-100%) to obtain compound 002-08.

LCMS: MS (ESI) m/z: 560.1[M+1]⁺.

### Step 8: Synthesis of compound 002-09

Compound 002-08 (160 mg, 285.91 µmol, 1 eq), a solid of ammonium chloride (15.29 mg, 285.91 µmol, 1 eq), iron powder (79.83 mg, 1.43 mmol, 5.0 eq), ethanol (4 mL) and water (2 mL) were added to a 10 mL bottle and stirred at 70 °C for 3 hours. The solid was removed by filtration, the reaction solution was concentrated, washed with 5 mL of dichloromethane, and then extracted with dichloromethane (10 mL×3), the organic phases were combined, dried with anhydrous sodium sulfate for half an hour, filtered, and concentrated to obtain a crude product compound 002-09.

LCMS: MS (ESI) m/z: 530.1[M+1]⁺.

### Step 9: Synthesis of compound 002-10

Compound 002-09 (151 mg, 285.10 µmol, 1.0 eq), compound 001-11 (76.36 mg, 342.12 µmol, 1.2 eq), diisopropylethylamine (151 mg, 285.10 µmol, 1.0 eq), dichloromethane (3 mL) and O-(7-azabenzotriazole-1-yl)-N,N,N,N-tetramethyl uronium hexafluorophosphate (162.61 mg, 427.65 µmol, 1.5 eq) were added to a 10 mL bottle and stirred at 40 °C for 3 hours. The reaction solution was concentrated, and 3 mL of methanol was added for HPLC separation to obtain compound 002-10 (column: Phenomenex Gemini-NX C18 75^{∗}30 mm^{∗}3 µm; mobile phase: [water (0.225% formic acid)-acetonitrile]; acetonitrile %: 50%-80%, 7 min).

LCMS: MS (ESI) m/z: 735.3[M+1]⁺.

### Step 10: Synthesis of compound 002

Compound 002-10 (70 mg, 95.26 µmol, 1 eq), methanol (0.2 mL), hydrogen chloride/dioxane (4 M, 23.82 µL, 1 eq) were added to a 50 mL bottle and stirred at 25 °C for 16 hours. The crude product was separated directly by high performance liquid chromatography to obtain the hydrochloride salt of the product 002 (separation conditions: column: Phenomenex Gemini-NX C18 75^{∗}30 mm^{∗}3 µm; mobile phase: [water (0.225% formic acid)-acetonitrile]; acetonitrile %: 20%-40%, 7 min).
LCMS: MS (ESI) m/z: 635.2[M+1]⁺;
¹H NMR (400 MHz, CD₃OD) δ ppm 1.67 (s, 4 H), 3.08 (s, 2 H), 4.15 (s, 2 H), 6.68 (d, J=5.52 Hz, 1 H), 7.09 (t, J=8.78 Hz, 2 H), 7.34 - 7.50 (m, 2 H), 7.58 (dd, J=8.91, 4.89 Hz, 2 H), 7.87 (dd, J=12.67, 2.13 Hz, 1 H), 8.01 (br d, J=8.03 Hz, 1 H), 8.07 - 8.27 (m, 2 H), 8.47 - 8.57 (m, 1 H), 8.68 (s, 1 H).

### Biological test data:

### Experimental Embodiment 1: Evaluation of kinase inhibitory activity in vitro

The ³³P isotope-labeled kinase activity assay (Reaction Biology Corp) was used to determine the IC₅₀ value to evaluate the inhibitory ability of the test compounds to Axl, c-Kit, Mer, DDR2, VEGFR1, VEGFR2, VEGFR3, TrkA, and FLT3.

Buffer conditions: 20 mM hydroxyethylpiperazine ethanethiosulfonic acid (Hepes) (pH 7.5), 10 mM MgCl₂, 1 mM ethylene glycol bisaminoethyl ether tetraacetic acid (EGTA), 0.02% polyoxyethylene dodecane Ether (Brij35), 0.02 mg/ml BSA, 0.1 mM Na₃VO₄, 2 mM dithiothreitol (DTT), 1% DMSO.

Compound Treatment: Test compounds were dissolved in 100% DMSO and diluted serially to a specific concentration with DMSO by Integra Viaflo Assist.

Test procedure: The substrate was dissolved in freshly prepared buffer, the tested kinase was added and gently mixed well. The DMSO solution containing the test compound was added to the above well mixed reaction solution using acoustic technique (Echo 550) and incubated at room temperature for 20 minutes. The compound concentrations in the reaction solution were 3 µM, 1 µM, 0.333 µM, 0.111 µM, 0.0370 µM, 0.0123 µM, 4.12 nM, 1.37 nM, 0.457 nM, 0.152 nM. After 15 minutes of incubation, ³³P-ATP (activity 0.01 µCi/µL, Km concentration) was added to start the reaction. The reaction was carried out at room temperature for 120 minutes, then radioactivity was detected by filter binding method. The kinase activity data were expressed by comparison between the kinase activity containing the test compound and the kinase activity of the blank group (containing DMSO only), and the IC₅₀ values were obtained by curve fitting using Prism4 software (GraphPad), and the experimental results are shown in Table 1.

**Table 1: Results of in vitro screening test for compounds of the present disclosure**

| Compound | IC₅₀ (nM) | | | | | | | | |
|---|---|---|---|---|---|---|---|---|---|
| | Axl | c-Kit | Mer | DDR2 | VEGFR1 | FLT3 | VEGFR3 | VEGFR2 | TrkA |
| Trifluoroacetate of 001 | 2.95 | 2.89 | 8.12 | 0.79 | 10.47 | 17.19 | 2.82 | 2.16 | 9.04 |
| Hydrochloride of 002 | 3.81 | 3.50 | 8.55 | 1.14 | 7.16 | 15.96 | 2.13 | 2.75 | 12.14 |

Conclusion: The compounds of the present disclosure exhibit good inhibitory activity on Axl, c-Kit, Mer, DDR2, VEGFR1, VEGFR2, VEGFR3, TrkA and FLT3.

### Experimental Embodiment 2: Evaluation of metabolic stability of liver microsomes in vitro

### 1. Materials

### 1.1 liver microsomes

SD rat, mouse, beagle, cynomolgus monkey and human microsomes were purchased from Corning, Xenotech or BioIVT and stored in a -80°C freezer.

### 1.2 Nicotinamide adenine dinucleotide phosphate, reduced (NADPH), supplier: Chem-impex international, catalog number: 00616.

### 1.3 Control compounds: testosterone, diclofenac, propafenone.

### 2. Experimental procedure

### 2.1 Preparation of working solution

Stock solution: 10 mM DMSO solution.

Working concentration preparation: 100% acetonitrile diluted to 100 µM (organic phase content: 99% ACN, 1% DMSO).

### 2.2 Experimental procedure

Prepare two 96-well incubation plates, which were named T60 incubation plate and NCF60 incubation plate, respectively.

445 µL of microsomal working solution (liver microsomal protein concentration of 0.56 mg/mL) was added to the T60 incubation plate and NCF60 incubation plate, respectively, and then the above incubation plates were preincubated in a 37°C water bath for about 10 minutes.

After the preincubation, 5 µL of test substance or working solution of control compound were added to the T60 incubation plate and NCF60 incubation plate, respectively, and mixed well. The reaction was initiated by adding 50 µL of potassium phosphate buffer to each well of the NCF60 incubation plate; 180 µL of termination solution (acetonitrile solution containing 200 ng/mL tolbutamide and 200 ng/mL labetalol) and 6 µL of NADPH regeneration system working solution were added to the T0 termination plate, and 54 µL of sample was removed from the T60 incubation plate to the T0 termination plate (T0 sample generation). The reaction was initiated by adding 44 µL NADPH regeneration system working solution to each well of the T60 incubation plate. 54 µL of microsomal working solution, 6 µL of NADPH regeneration system working solution and 180 µL of termination solution were added only to the blank plate. Therefore, in the samples of the test or control compounds, the final reaction concentration of compound, testosterone, diclofenac and propafenone was 1 µM, the concentration of liver microsomes was 0.5 mg/mL, and the final concentration of DMSO and acetonitrile in the reaction system were 0.01% (v/v) and 0.99% (v/v), respectively.

After incubation for an appropriate time (e.g. 5, 10, 20, 30 and 60 minutes), 180 µL of termination solution (acetonitrile solution containing 200 ng/mL tolbutamide and 200 ng/mL labetalol) was added to the sample well of each termination plate, respectively, and then 60 µL of sample was removed from the T60 incubation plate to terminate the reaction.

All sample plates were shaken well and centrifuged at 3220 × g for 20 minutes, then 80 µL of supernatant per well was diluted into 240 µL of purified water for liquid chromatography tandem mass spectrometry analysis.

### 3. Liquid chromatography tandem mass spectrometry analysis

All samples were injected for analysis.

The experimental results are shown in Table 2.

**Table 2: Results of in vitro liver particle metabolism stability test for compounds of the present disclosure**

| Species | Parameter | Sitravatinib | Trifluoroacetate of 001 | Hydrochloride of 002 |
|---|---|---|---|---|
| Human | CLᵢₙₜ₍ₗᵢᵥₑᵣ₎ (mL/min/kg) | 10.6 | <8.6 | <8.6 |
| | 60 min remaining | 71.5% | 87.1% | 96.4% |
| Mouse | CLᵢₙₜ₍ₗᵢᵥₑᵣ₎ (mL/min/kg) | 460.4 | 321 | 301 |
| | 60 min remaining | 3.30% | 9.5% | 11.3% |
| Rat | CLᵢₙₜ₍ₗᵢᵥₑᵣ₎ (mL/min/kg) | 191.8 | 71.3 | 78.1 |
| | 60 min remaining | 4.2% | 28.6% | 26.2% |
| Dog | CLᵢₙₜ₍ₗᵢᵥₑᵣ₎ (mL/min/kg) | 172.5 | 144.5 | 139.4 |
| | 60 min remaining | 2.6% | 4.9% | 5.4% |
| Monkey | CLᵢₙₜ₍ₗᵢᵥₑᵣ₎ (mL/min/kg) | 42.8 | 22.5 | 19.7 |
| | 60 min remaining | 38.5% | 55.9% | 59.7% |

| | | | | |
|---|---|---|---|---|
| Note: CL_{int (liver)} : hepatic intrinsic clearance. | | | | |

Conclusion: The compounds of the present disclosure exhibits better stability than the reference compound sitravatinib.

### Experimental Embodiment 3: Pharmacokinetic evaluation of compounds

Experimental purpose:
Testing the pharmacokinetic properties of the compounds of the present disclosure in rats.

Experimental Materials:
SD rat (male)

Experimental operation:
The pharmacokinetic characteristics of the compounds in rodents after oral administration were tested by standard protocols. In the experiment, the candidate compounds were formulated into a clarified solution and given to rats for oral administration. The vehicle was 10% NMP/40% PEG400/50%. Whole blood samples were collected within 24 hours (0.0833, 0.25, 0.5, 1, 2, 4, 6, 8, 24 h), and all blood samples were added to the plastic centrifuge tubes pre-labeled with 0.5 M K2-EDTA anticoagulant. After blood samples collection, the supernatant plasma was adsorbed after centrifuging at 4°C, 3000 xg for 10 minutes and quickly placed in dry ice at -20°C or lower, blood concentrations were quantified analyzed by LC-MS/MS analysis method and pharmacokinetic parameters such as peak concentration, time to peak, clearance rat, half-life, area under the drug time curve, bioavailability, etc. were calculated.

Experimental results:

**Table 3: Pharmacokinetic test results in rats**

| Test sample (compound) | Oral administration | | | |
|---|---|---|---|---|
| | Administration dose (mpk) | Cₘₐₓ (nM) | Tₘₐₓ(h) | AUC₀₋ₗₐₛₜ (nM.hr) |
| Sitravatinib | 5 | 801 | 3.0 | 4794 |
| Trifluoroacetate of 001 | 5 | 930 | 1.25 | 6650 |

| | | | | |
|---|---|---|---|---|
| Note: Cₘₐₓ: peak concentration; Tₘₐₓ: peak time ; AUC₀₋ₗₐₛₜ: area under the plasma concentration-time curve from the zero time point to the last detectable concentration time point. | | | | |

Conclusions: The compound of the present disclosure exhibits good pharmacokinetic indices in rats and the exposure of compound 001 was superior to the reference compound sitravatinib.

## Claims

1. A compound represented by formula (I) or a pharmaceutically acceptable salt thereof, 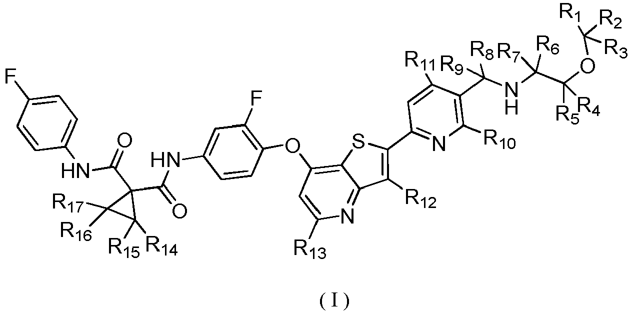 wherein,
R₁, R₂, R₃, R₄, R₅, R₆, R₇, R₈, R₉, R₁₀, R₁₁, R₁₂, R₁₃, R₁₄, R₁₅, R₁₆ and R₁₇ are each independently selected from H and D, and at least one of them is D.

2. The compound or the pharmaceutically acceptable salt thereof as defined in claim 1, wherein, R₁, R₂ and R₃ are each independently selected from H and D.

3. The compound or the pharmaceutically acceptable salt thereof as defined in claim 1 or 2, wherein, R₄ and R₅ are each independently selected from H and D.

4. The compound or the pharmaceutically acceptable salt thereof as defined in claim 1 or 2, wherein, R₆ and R₇ are each independently selected from H and D.

5. The compound or the pharmaceutically acceptable salt thereof as defined in claim 1 or 2, wherein, R₈ and R₉ are each independently selected from H and D.

6. The compound or the pharmaceutically acceptable salt thereof as defined in claim 1 or 2, wherein, R₁₀ is selected from H and D.

7. The compound or the pharmaceutically acceptable salt thereof as defined in claim 1 or 2, wherein, R₁₁ is selected from H and D.

8. The compound or the pharmaceutically acceptable salt thereof as defined in claim 1 or 2, wherein, R₁₂ is selected from H and D.

9. The compound or the pharmaceutically acceptable salt thereof as defined in claim 1 or 2, wherein, R₁₃ is selected from H and D.

10. The compound or the pharmaceutically acceptable salt thereof as defined in claim 1 or 2, wherein, R₁₄, R₁₅, R₁₆ and R₁₇ are each independently selected from H and D.

11. A compound or a pharmaceutically acceptable salt thereof:

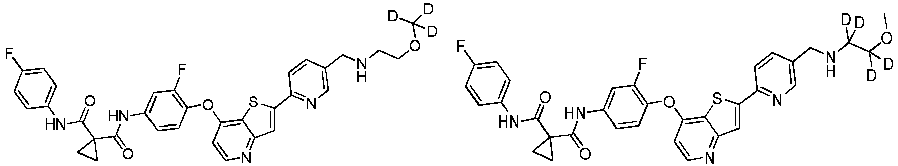

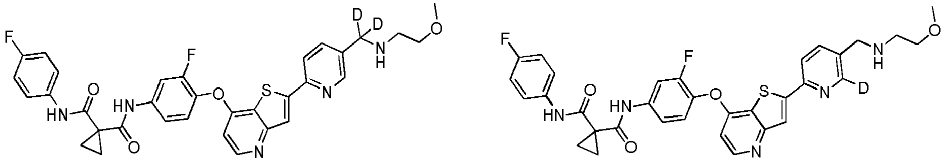

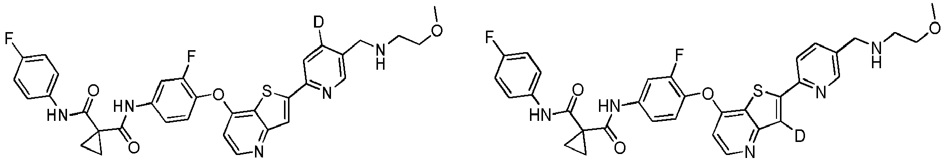

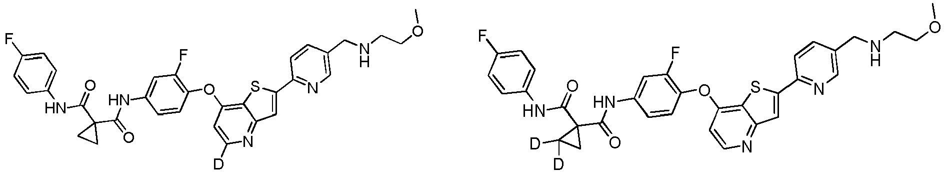

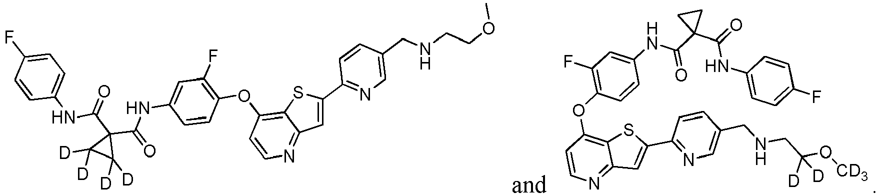

12. Use of the compound or the pharmaceutically acceptable salt according to any one of claims 1 to 11 in the manufacture of a medicament for diseases related to tyrosine kinase.
